# EUROPEAN PATENT SPECIFICATION

(11) **EP 3 551 108 B1**
(45) Date of publication and mention of the grant of the patent: **22.06.2022**
(21) Application number: 18751211.6
(22) Date of filing: 09.02.2018
(51) Int. Cl.: A61B 18/00, A61B 18/02, A61B 18/14

(54) **APPARATUS AND METHOD FOR CRYOABLATION**
VORRICHTUNG UND VERFAHREN ZUR KRYOABLATION
APPAREIL ET PROCÉDÉ POUR CRYOABLATION

(30) Priority: 10.02.2017 US 201762457666 P
(43) Date of publication of application: 16.10.2019
(73) Proprietor: St. Jude Medical, Cardiology Division, Inc., St. Paul, MN 55117 (US)
(72) Inventor: HOU, Wenbo, Santa Clarita California 91350 (US); MIN, Xiaoyi, Camarillo California 93012 (US)
(74) Representative: Kramer Barske Schmidtchen Patentanwälte PartG mbB
(86) International application number: PCT/US2018/017645
(87) International publication number: WO 2018/148561

(56) References cited:
- EP-A1- 1 464 296
- EP-A1- 1 464 296
- EP-A2- 1 502 554
- US-A- 5 423 807
- US-A1- 2006 100 495
- US-A1- 2009 043 186
- US-A1- 2009 287 202
- US-A1- 2009 287 202
- US-A1- 2012 029 512
- US-A1- 2012 065 631
- US-A1- 2012 065 631
- US-A1- 2012 109 118
- US-A1- 2014 052 118

## Description

### a. Field

This disclosure relates to apparatuses and methods for ablating tissue. In particular, the instant disclosure relates to using a flexible catheter to ablate multiple locations.

### b. Background Art

US 2009/0287202 A1 discloses an apparatus for cryogenically ablating tissue.

The human heart routinely experiences electrical impulses traversing its many surfaces and chambers, including the endocardial chamber. The heart depolarizes and repolarizes, as electrical currents spread across the heart and throughout the body. In healthy hearts, the atria and ventricles of the heart will experience an orderly progression of depolarization waves. In unhealthy hearts, such as those experiencing atrial arrhythmia, including for example, ectopic atrial tachycardia, atrial fibrillation, and atrial flutter, the progression of the depolarization waves becomes chaotic. Arrhythmias may persist as a result of scar tissue or other obstacles to rapid and uniform depolarization. These obstacles may cause depolarization waves to electrically circulate through some parts of the heart more than once. Atrial arrhythmia can create a variety of dangerous conditions, including irregular heart rates, loss of synchronous atrioventricular contractions, and blood flow stasis. All of these conditions have been associated with a variety of ailments, including death.

Catheters are used in a variety of diagnostic and/or therapeutic medical procedures to correct conditions such as atrial arrhythmia, including for example, ectopic atrial tachycardia, atrial fibrillation, and atrial flutter.

Typically in a procedure, a catheter is manipulated through a patient's vasculature to, for example, a patient's heart, and carries one or more electrodes which may be used for mapping, ablation, diagnosis, or other procedures. Where an ablation therapy is desired to alleviate symptoms including atrial arrhythmia, an ablation catheter imparts ablative energy to cardiac tissue to create a lesion in the cardiac tissue. The lesioned tissue is less capable of conducting electrical impulses, thereby disrupting undesirable electrical pathways and limiting or preventing stray electrical impulses that lead to arrhythmias. The ablation catheter may utilize ablative energy including, for example, radio frequency (RF), cryoablation, laser, chemical, and high-intensity focused ultrasound. As readily apparent, such an ablation treatment requires precise positioning of the ablation catheter for optimal results.

Typically, ablation therapies have been delivered by making a number of individual lesions in a controlled fashion in order to form a lesion line. Such lesion lines are often desirable around/between the pulmonary veins in the left atrium of the heart which have been associated with the introduction of erratic electric impulses into the heart. There are devices in development or being commercialized that attempt to achieve a sufficient lesion line with minimal applications of energy. Existing designs range from diagnostic catheters and balloon mounted designs with energy applying features. The existing designs often suffer from a lack of continuous contact around a circumference of the pulmonary vein during therapy delivery, resulting in inconsistent lesion lines and incomplete electrical impulse blockage.

The foregoing discussion is intended only to illustrate the present field and should not be taken as disavowal of claim scope.

### BRIEF SUMMARY

The instant disclosure, in at least one embodiment, provides an apparatus for providing therapy to tissue comprising a flexible shaft with a distal end and a proximal end, and a planar therapy structure, where the planar therapy structure is coupled with the distal end of the flexible shaft, where the planar therapy structure comprises an inlet to receive a pressurized coolant from a supply line, a plurality of openings to provide the pressurized coolant to an expansion chamber, and an outlet to receive a de-pressurized coolant from the expansion chamber.

In another embodiment, a system comprises a coolant source, wherein the coolant source generates a pressurized coolant, a coolant control, wherein the coolant control controls the coolant source and the pressurized coolant, a catheter, wherein the catheter is coupled with the coolant source and the catheter comprises a planar therapy structure, where the planar therapy structure is coupled with the distal end of the shaft, where the planar therapy structure comprises an inlet to receive the pressurized coolant from the coolant source, a plurality of openings to provide the pressurized coolant to an expansion chamber, wherein the inlet and the outlet pass through an expansion chamber end wall at a proximal end of the expansion chamber and the plurality of openings are coupled with the inlet and are inside the expansion chamber, and an outlet to receive a de-pressurized coolant from the expansion chamber.

In yet another embodiment not part of the invention, a method for cooling tissue, comprises circulating a coolant through a portion of the catheter, wherein the catheter has a planar structure at a distal end portion, inputting the coolant through an inlet, dispensing the coolant from the inlet through a plurality of openings into an expansion chamber at the distal end portion of the catheter, cooling a tissue proximate the distal end portion of the catheter, outputting the coolant from the expansion chamber through an outlet, wherein a coolant supply line is coupled with the inlet, and the inlet is coupled with the plurality of openings and the outlet is coupled with the expansion chamber.

### BRIEF DESCRIPTION OF THE DRAWINGS

FIG. 1 is a system diagram showing a medical device and an energy/fluid supply, in accordance with embodiments of the present disclosure.
FIG. 2 is a system diagram showing an exemplary cryoablation catheter system, consistent with embodiments of the present disclosure.
FIG. 3A is a schematic view of a cryoablation catheter system that includes a cryoablation catheter, a coolant control, and a pressure system, consistent with embodiments of the present disclosure.
FIG. 3B is a side view of a distal end of the cryoablation catheter of FIG. 3A contacting tissue, consistent with embodiments of the present disclosure.
FIG. 3C is a cross-sectional view along line 3C-3C of the catheter of FIGS. 3A and 3B, consistent with embodiments of the present disclosure.
FIG. 4A is a cross-sectional view of a distal end of a cryoablation catheter, consistent with embodiments of the present disclosure.
FIG. 4B is a cross-sectional view of a portion of the catheter wall and the electrode of FIG. 4A, consistent with embodiments of the present disclosure.
FIG. 5A is a cross-sectional view of the distal end of the inlet tube and the plurality of openings of FIG. 4A, consistent with embodiments of the present disclosure.
FIG. 5B is a cross-sectional view of a plurality of openings similar to FIG. 4A where each of the plurality of openings receive coolant from a separate supply line, consistent with embodiments of the present disclosure.
FIG. 5C is a cross-sectional view of the plurality of openings of FIG. 4A where the plurality of openings receive coolant from a manifold that receives coolant input from a single supply line and outputs to each of the plurality of openings separately, consistent with embodiments of the present disclosure.
FIG. 6 is a cross-sectional view of a cryoablation catheter, consistent with embodiments of the present disclosure.
FIG. 7A is an isometric view of a cryoablation catheter with a hoop at a distal end, consistent with embodiments of the present disclosure.
FIG. 7B is an isometric view of a cryoablation catheter with multiple hoops at a distal end, consistent with embodiments of the present disclosure.
FIG. 7C is a cross-sectional front-view of a left atrium with the cryoablation catheter with multiple hoops of FIG. 7B positioned proximate a pulmonary vein in contact with tissue, consistent with various aspects of the present disclosure.
FIG. 8 is a schematic diagram of a cryoablation system, consistent with embodiments of the present disclosure.
FIG. 9 shows an exemplary method for circulating coolant through a portion of a catheter to cool tissue, consistent with unclaimed embodiments of the present disclosure.

### DETAILED DESCRIPTION OF EMBODIMENTS

The instant disclosure relates to catheters for tissue ablation, and more specifically, electrophysiology catheters within the heart. In particular, the instant disclosure relates to a catheter that conforms a portion of tissue to a shape and/or an electrophysiology catheter that conforms to a shape of a pulmonary vein. The pulmonary vein can receive therapy for cardiac arrhythmias and the tissue ablation can produce a consistent tissue ablation line along a length and circumference of the pulmonary venous tissue. Details of the various embodiments of the present disclosure are described below with specific reference to the figures.

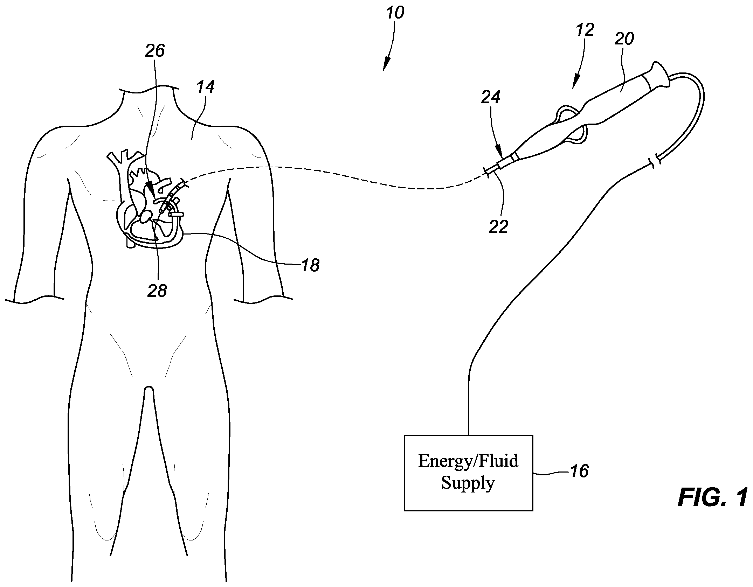

Referring now to the drawings wherein like reference numerals are used to identify similar components in the various views, FIG. 1 is a system diagram showing a medical device and an energy/fluid supply, in accordance with embodiments of the present disclosure. In some embodiments, and with reference to FIG. 1, the system 10 can include a medical device 12 and an energy/fluid supply 16 (e.g., an RF ablation generator, a coolant supply, etc.). In an embodiment, the catheter 12 may be an ablation catheter. The catheter 12 can be configured to be inserted into a patient's heart 18.

The catheter 12 may include a handle 20 and a shaft 22 having a proximal end portion 24, a distal end portion 26, and a tip portion 28 disposed at the distal end portion 26 of the shaft 22. The catheter 12 may further include other conventional components such as, for example and without limitation, a temperature sensor, a position sensor, additional sensors or electrodes, and corresponding conductors or leads. For purposes of illustration and clarity, the description below will be limited to an embodiment wherein the medical device 12 comprises a catheter (a sample catheter is shown in FIG. 1 (e.g., catheter 12)). It will be appreciated, however, that the present disclosure is not meant to be limited to catheters

The shaft 22 can be an elongate, tubular, flexible member configured for movement within the body 14. The tip portion 28 of the shaft 22 supports, for example and without limitation, sensors and/or electrodes mounted thereon. The tip portion 28 may include ablation elements (e.g., ablation tip electrodes for delivering RF ablative energy). The shaft 22 may also permit transport, delivery, and/or removal of fluids (including irrigation fluids, cryogenic ablation fluids, and bodily fluids), medicines, and/or surgical tools or instruments.

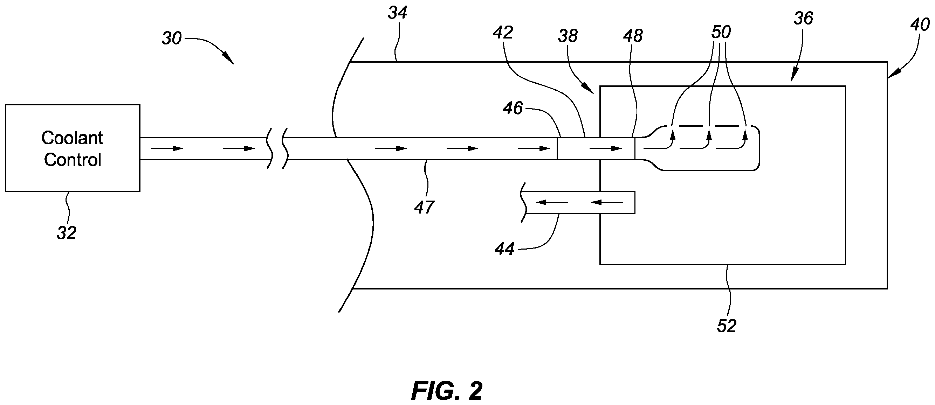

FIG. 2 is a system diagram showing an exemplary cryoablation catheter system, consistent with embodiments of the present disclosure. The cryoablation catheter system 30 can include a coolant control 32, an elongate medical device 34 (e.g., a catheter), and an expansion chamber 36. The catheter 34 can include a proximal end 38 and a distal end 40, where the distal end 40 can be coupled with the expansion chamber 36 and the proximal end 38 can be coupled with the coolant control 32 The expansion chamber 36 can include an inlet 42 and an outlet 44.

The coolant control 32, the catheter 34, the expansion chamber 36, the inlet 42, and the outlet 44 can be coupled to form a circulation loop. The circulation loop can circulate a coolant (e.g., a fluid or a gas) where the circulation is controlled by the coolant control 32. The coolant control 32 can control and monitor, for example, a rate of flow of the coolant and a pressure of the coolant. A proximal end 46 of the inlet 42 can be coupled with the coolant control 32 (e.g., by a tube 47). A distal end 48 of the inlet 42 can include a plurality of openings 50 where the plurality of openings 50 are inside the expansion chamber 36. The coolant can be circulated from the coolant control 32 through the inlet 42 to the plurality of openings 50. The plurality of openings 50 can allow the coolant to be directed into the expansion chamber 36. The coolant can flow out of the expansion chamber 36 through the outlet 44. The circulation loop can be a closed loop (e.g., the coolant is reused after leaving the expansion chamber 36) or the circulation loop can be open (e.g., the coolant is not reused after leaving the expansion chamber 36).

As the coolant is circulated into the expansion chamber 36 through the plurality of openings 50, an outer wall 52 of the expansion chamber 36 can be cooled. The cooling of the outer wall 52 can create a temperature gradient (e.g., if a first temperature outside the expansion chamber is higher than a temperature at the outer wall 52 of the expansion chamber 36) between the expansion chamber 36 and the area proximate the outer wall 52. In embodiments utilizing cryogenic ablation methodologies, super-cooled fluid for ablating tissue (e.g., pulmonary venous tissue) can be pumped into the expansion chamber 36 and ablate the tissue in contact with the catheter 34 (and in some cases in proximity therewith).

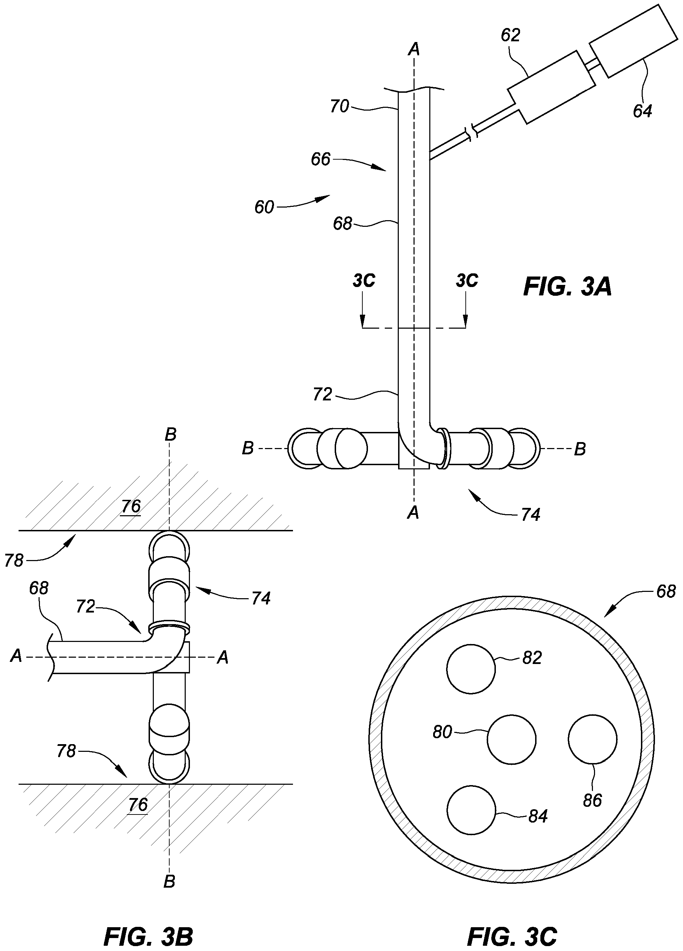

FIG. 3A is a schematic view of a cryoablation catheter system that includes a cryoablation catheter, a coolant control, and a pressure system, consistent with embodiments of the present disclosure. The cryoablation catheter system 60 can include a coolant control 62 and the pressure system 64, where the coolant control can be coupled to the cryoablation catheter system 60. In some embodiments, the pressure system 64 can be coupled with the catheter system 60. The coolant control 62 and the pressure system 64 can be coupled with, for example, a proximal end portion 66 of a catheter 68 with a longitudinal axis defined by the line A-A. In some embodiments, the coolant control 62 and the pressure system 64 can be coupled with catheter 68 at a location between a proximal end 70 and the distal end 72 of the catheter 68. In some embodiments, the coolant control 62 and the pressure system 64 can be connected in series as shown in FIG. 3A or, in other embodiments, the coolant control 62 and the pressure system 64 can each be separately connected to the catheter 68 at the same location or different locations. The pressure system 64 can, for example, pressurize a coolant (e.g., a fluid or a gas) in the cryoablation catheter system 60.

The coolant control 62 can, for example, control and monitor a rate of flow of the coolant and a pressure of the coolant. The coolant control 62 can be connected to the catheter 68 at any suitable location (e.g., proximate the proximal end 70 or some other location along the length of the catheter 68). In some embodiments, the coolant control 62 can include a coolant reservoir (not shown). In other embodiments, the coolant reservoir can be separate from the coolant control 62.

The distal end 72 of the catheter 68 can be formed into a hoop 74 or other shape (oval, square, etc.) to facilitate contact between the hoop 74 of the catheter 68 and tissue 76 (not shown in FIG. 3A, see FIG. 3B). The hoop 74 can be configured to form a planar structure aligned with the plane BB (represented by line BB in FIG. 3A). An angle of the plane BB of the hoop 74 can be perpendicular to the longitudinal axis AA of the catheter. In other embodiments, the angle between the line AA and plane BB can be something other than perpendicular to the longitudinal axis of the catheter (e.g., 45°, 60°, etc.). The angle can be adjustable or fixed during use.

FIG. 3B is a side view of a distal end of the cryoablation catheter of FIG. 3A contacting tissue, consistent with embodiments of the present disclosure. The catheter 68 can include the hoop 74. The hoop 74 can be sized to fit, for example, into a wide antral portion of a pulmonary vein (PV) 78. In other embodiments, the hoop 74 can be sized (e.g., smaller diameters) to fit further into the sleeve of the PV 78 past the wider antral portion near the opening of the PV 78. The hoop 74 can include diameters ranging between, for example, 12-33 mm. The distal end 72 of the catheter 68 with the hoop 74 can be maneuvered adjacent to the PV 78 using any suitable method and the hoop 74 can be maneuvered, by moving the catheter 68, to a location of the PV 78.

As a result of the hoop 74 being positioned in the PV 78, the hoop 74 can contact the tissue 76 of the PV 78. The hoop 74 can be sized and/or placed so that it re-models the PV (e.g., stretches the tissue at that location of the PV 78, but only in a temporary manner). The re-modeling of the PV 78 can be caused, for example, by the compliancy of the PV 78 compared to the structure of the hoop 74. In other embodiments, the hoop 74 can be used to contact tissue in any suitable location of a body (e.g., locations in addition to the PV). The hoop 74 can have different sizes and configurations to be configured for use with various locations of the body (e.g., linear configuration to contact tissue in a generally planar manner, etc.).

FIG. 3C is a cross-sectional view along line 3C-3C of the catheter of FIGS. 3A and 3B, consistent with embodiments of the present disclosure. The catheter 68 at location 3C of FIG. 3A can include a central lumen 80, a lumen for wires 82, a first tube 84 for inflow, and a second tube 86 for outflow.

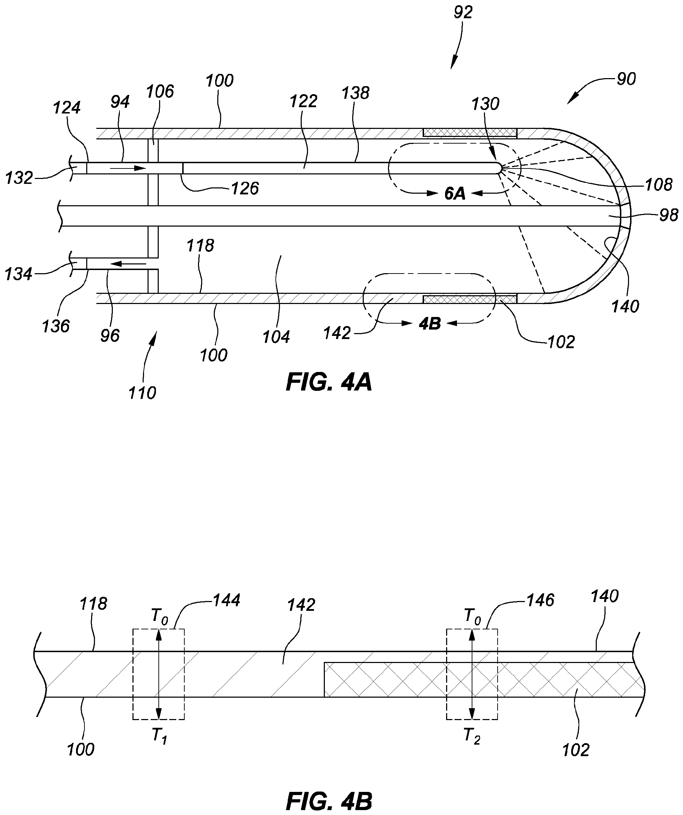

The central lumen 80 can be used to, for example, provide a location for a guidewire. A guidewire can be used to maneuver the catheter 68 to a therapy location in a body. In still other embodiments, there can be a central lumen 80 for some portion of the catheter 68 and then the central lumen 80 can exit the catheter 68 at a location other than the distal tip (e.g., a neck location, not shown). The lumen for wires 82 can be used for providing wires, cables, or other similar devices along the length of the catheter 68. For example, wires for sensors, electrodes, valves, nozzles or other similar devices can be located in the lumen for wires 82. The first tube 84 for inflow can be connected to, for example, a coolant control and an inlet for an expansion chamber (not shown in FIG. 3C, see, e.g., FIG. 4A). The second tube 86 for outflow can be used to remove or exhaust coolant from the expansion chamber.

Some embodiments of the catheter 68 of FIGS. 3A-3C may not have a central lumen 80 as other methods may be used to maneuver the catheter 68 to the therapy location. In the embodiments without a central lumen 80, the catheter 68 can be delivered to a therapy site as a stand-alone device, using an introducer or other similar device.

FIG. 4A is a cross-sectional view of a distal end of a cryoablation catheter, consistent with embodiments of the present disclosure. The distal end 90 of the cryoablation catheter 92 can include an inlet 94, an outlet 96, a central lumen 98, an exterior wall 100, an electrode 102, an expansion chamber 104, an expansion chamber end wall 106, and a plurality of openings 108.

The expansion chamber 104 can be coupled with a distal end portion 110 of the catheter 92 and include the expansion chamber end wall 106 that spans the diameter of the interior of the catheter 92. The expansion chamber end wall 106 can include a variety of openings. For example, the inlet 94, the outlet 96, and the central lumen 98 can pass through the expansion chamber end wall 106. In some embodiments, the expansion chamber end wall 106 may only include the inlet 94 and the outlet 96 and omit the central lumen 98. The size of the expansion chamber 104 can be any suitable size as the expansion chamber wall 106 can be placed at any suitable location of the distal end portion 110 of the catheter 92. In some embodiments, more than one expansion chamber can be included (not shown). If multiple expansion chambers are used, each can have one or more of the plurality of openings 108 providing coolant and an outlet into, for example, another expansion chamber (e.g., an adjacent chamber) or to a discharge line that is connected to the outlet in the expansion chamber wall.

The expansion chamber end wall 106 can be coupled with an interior surface 118 of the catheter 92. The expansion chamber end wall 106 can include the inlet 94 and the outlet 96 In other embodiments, the expansion chamber end wall 106 can include additional inlet and outlets. The expansion chamber end wall 106 can be located proximate the distal end 90 of the catheter 92.

In some embodiments, the expansion chamber end wall 106 can be positioned so the expansion chamber 104 can be a length sufficient to permit the formation of a hoop, loop, or other shape at the distal end 90 of the catheter 92 for contact with tissue (e.g., contact with a PV or other tissue). In other embodiments, the expansion chamber end wall 106 can be located in other portions of the catheter 92 (e.g., near the center point of the length of catheter 92, proximate the proximal end (not shown in FIG. 4A) of the catheter 92, etc.). The expansion chamber end wall 106 can be made of any suitable material (e.g., a polymer, a metal, etc.). In some embodiments, the wall of the expansion chamber 106 is the catheter 92 wall (e.g., the outer wall 100 is part of the catheter 92 wall). In other embodiments, the wall of the expansion chamber 106 and the wall of the catheter 92 are different and separate walls (e.g., the wall of the expansion chamber 106 is in addition to the wall of the catheter 92).

The inlet 94 can be an opening through the expansion chamber end wall 106 to facilitate input of a coolant (e.g., a fluid or a gas). The inlet 94 can be coupled with an inlet tube 122. The inlet 94 and inlet tube 122 can be a single element where a proximal end 124 of the inlet 94 is configured to receive other connective elements (e.g., tubes, hoses, pipes, fittings, etc.). The inlet 94 can have a distal end 126 that can be coupled with the a proximal end 124 of the inlet tube 122. The inlet tube 122 can have a distal end 130. In some embodiments, the inlet can be a single element that includes a length of tubing, a hose, or a pipe. The inlet 94 can be coupled with a supply line 132 on the proximal end 124 of the inlet 94 (e.g., the side that is exterior of the expansion chamber 104). In some embodiments, more than one inlet can be used. The inlet tube 122 can include the plurality of openings 108 (discussed in greater detail below). In some embodiments, the supply line 132 can pass through the inlet 94 and continue into the inlet tube 122 and, for example, connect (directly or indirectly) to the plurality of openings 108).

The outlet 96 can be an opening through the expansion chamber end wall 106 to facilitate discharge or removal of the coolant (e.g., a fluid or a gas) from the expansion chamber 104. The outlet 96 can be a single element configured to receive other connective elements (e.g., tubes, hoses, pipes, fittings, etc.). In some embodiments, the outlet 96 can be a single element that includes a length of tubing, a hose, or a pipe. The outlet 96 can be coupled with, for example, a discharge line 134 on a proximal end 136 of the outlet 96 (e.g., the side that is exterior of the expansion chamber 104). In some embodiments, more than one outlet can be used.

The plurality of openings 108 can be openings in a wall 138 of the inlet tube 122. The plurality of openings 108 can be distributed along the length of the inlet tube 122 with any suitable spacing. The plurality of openings 108 can also be spaced radially around the inlet tube 122. The plurality of openings 108 can be positioned to provide various patterns of discharge of the coolant into the expansion chamber 104. For example, the plurality of openings 108 can be placed so that the coolant flows towards an interior surface 140 of the expansion chamber 104. The plurality of openings 108 can be, for example. the same size or they can be different sizes or any suitable combination of sizes. The plurality of openings 108 can also provide directional control when the coolant is discharged into the expansion chamber 104. In some embodiments, the plurality of openings 108 can also include a nozzle on one or more of the openings (not shown). The nozzle can be used to control or direct the flow of the coolant. For example, the nozzle can be configured to direct the coolant to a specific location/area of the expansion chamber 104 or to discharge the coolant in a specific pattern.

The plurality of openings 108 can include, for example, groups of openings (not shown) at different locations along the length of the inlet tube 122. For example, a first group of openings can be spaced radially around a first location on the inlet tube 122 and a second group of openings can be spaced radially around a second location on the inlet tube 122. Any suitable number of groups of openings can be used and the number of openings in each group can be the same or different. The sizes of the each opening in the various groups can be the same or different.

The plurality of openings 108 can be arranged to allow for a variation in a pressure of the coolant as the coolant is discharged through the plurality of openings 108. For example, the opening that is most proximal (e.g., closest to the inlet) can have a higher pressure of the coolant and the opening that is most distal (e.g., furthest from the inlet) can have a lower pressure of the coolant. To help minimize the difference in the pressure and/or amount of the coolant being discharged from each of the plurality of openings 108, each of the plurality of openings 108 can, for example, be a different size as you move from the inlet proximal end 128 towards the distal end 126 of the inlet tube 122. For example, the most proximal opening can be the smallest opening and the most distal opening can have the largest opening, with incremental changes in opening size in between.

The electrode 102 can be proximate the distal end 90 of the catheter 92. The electrode 102 can be any suitable type of electrode (ring electrode, etc.). In some embodiments, multiple electrodes can be used. The electrode 102 can be used for navigation, sensing, therapy (e.g., RF ablation), or other uses. In some embodiments, two electrodes can be located proximal to the distal end 90 of the catheter 92 and generate overlapping signals used in sensing and/or therapy (discussed in greater detail below). In some embodiments, an electrode (e.g., the electrode 102) can be cooled by the coolant and the electrode can be a focal point for cooling tissue adjacent to the electrode. For example, the electrode, after being cooled by the coolant, can provide treatment to tissue (e.g., create a lesion). The electrodes can be spaced such that the lesions generated overlap, and/or are close enough to provide treatment to tissue for various therapies.

The catheter 92 can be made from any suitable material (e.g., polyurethane, etc.). The material can permit the catheter 92 to bend and/or flex during use to form various shapes and configurations. The catheter 92 can have walls portions that are made from a single type of material or the walls can have portions that include multiple combinations of different materials (different types of polymers, metals, ceramics, etc.). For example, a wall 142 can have a first material on the interior wall 118 and a second material on the exterior wall 100. The various materials and/or layers can be continuous or non-continuous. In some embodiments the thickness of the wall of the catheter 92 can vary (e.g., adjacent an electrode or other sensor).

FIG. 4B is a cross-sectional view of a portion of the catheter wall and the electrode of FIG. 4A, consistent with embodiments of the present disclosure. As described above, as the coolant is circulated into the expansion chamber 104 through the plurality of openings 108, a wall 142 of the expansion chamber 104 can be cooled (e.g., in an embodiment where the expansion chamber uses the wall 100 of the catheter as the expansion chamber wall), which can also cool the adjacent wall of the catheter 92 (in an embodiment (not shown) where the expansion chamber wall is separate and in addition to the wall 100 of the catheter 92). As described above, in some embodiments, the catheter wall is the same as the expansion chamber wall.

A temperature, T, in the expansion chamber 104 can be T₀. The temperature outside the catheter 92 can be T₁ at wall section 144 of the catheter 92 and T₂ at the electrode section 146 of the catheter 92. This can create a temperature gradient between the expansion chamber 104 and the exterior of the catheter 92. The temperature gradient can vary or be the same along the length of expansion chamber 104. For example, a thermal property of an area of the catheter 92 with just the catheter 92 and the expansion chamber 104 can differ from a thermal property of an area of the catheter that includes the electrode 102. In that example, a first temperature gradient at the wall section 144 of the expansion chamber 104 can be a difference between T₀ and T₁ and a second temperature gradient at the wall section 146 of the expansion chamber 104 can be a difference between T₀ and T₂. The materials used in the catheter can be selected to, for example, minimize the differences in temperature gradients at different locations (e.g., areas with electrodes and areas without electrodes).

In some embodiments, a consistent exterior wall temperature may be desired (e.g., for T₁ to equal T₂ for even cryoablation of tissue) at the distal end portion 110 of the catheter 92. To help achieve this, the plurality of openings 108 can be varied, for example, in number, location, directionality, and size, to account for varying thermal properties of the expansion chamber 104 and/or the catheter 92 and to limit and/or eliminate effects of different temperature gradients through portions of the walls of the expansion chamber 104 and/or the catheter 92. In this embodiments, an amount of coolant may be sufficient to cool the expansion chamber 104 so that all locations of the wall 142 are configured to provide treatment (e.g., cryoablation).

In some embodiments, a different exterior wall temperature may be desired (e.g., for T₁ to not equal T₂ for concentrated cryoablation of tissue at specific locations). To help achieve this, the plurality of openings 108 can be specific, for example, in number, location, directionality, and size, to account for varying thermal properties of the expansion chamber 104 and/or the catheter 92 and to concentrate effects of different temperature gradients through specific portions of the walls of the expansion chamber 104 and/or the catheter 92.

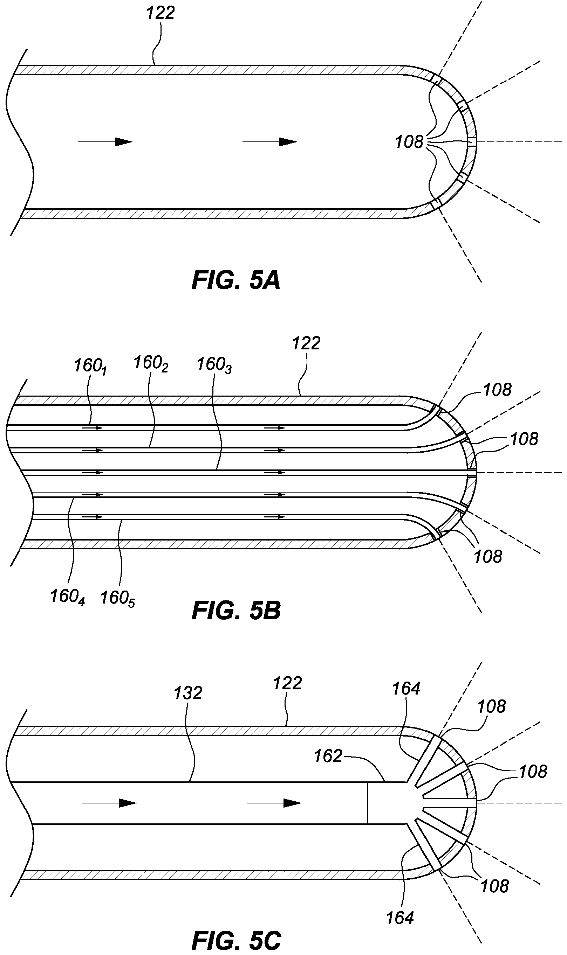

FIG. 5A is a cross-sectional view of the distal end of the inlet tube and the plurality of openings of FIG. 4A, consistent with embodiments of the present disclosure. The inlet tube 122 can include the plurality of openings 108. The supply line 132 (from FIG. 4A) can provide a coolant to a plurality of openings 108. The supply line 132 can be connected to a coolant control and/or a coolant reservoir (not shown). For example, the distal end 126 of the inlet 94 (shown in FIG. 4A) can be coupled with the supply line 132. The inlet 94 can be coupled with the proximal end 124 of the inlet tube 122. FIG. 5A is a close-up view of the distal end 126 of the inlet tube 122 and the plurality of openings 108. As shown in FIG. 5A, the plurality of openings 108 can receive the coolant through a supply line (e.g., the supply line 132 shown in FIG. 4A) to discharge the coolant from the plurality of openings 108 into the expansion chamber 104 (as shown in FIG. 4A).

FIG. 5B is a cross-sectional view of a plurality of openings similar to FIG. 4A where each of the plurality of openings receive coolant from a separate supply line, consistent with embodiments of the present disclosure. The inlet tube 122 can include a plurality of openings 108 where each can be connected to a corresponding supply line 160₁, 160₂, 160₃, 160₄, and 160₅. The supply lines 160₁, 160₂, 160₃, 160₄, and 160₅ can be connected to the coolant control and/or a coolant reservoir (not shown).

FIG. 5C is a cross-sectional view of the plurality of openings of FIG. 4A where the plurality of openings receive coolant from a manifold that receives coolant input from a single supply line and outputs to each of the plurality of openings separately, consistent with embodiments of the present disclosure. The coolant control and/or a coolant reservoir (not shown) can be connected to a manifold 162 by the supply line 132. The manifold 162 can include a plurality of manifold openings 164 to correspond to each of the plurality of openings 108 of the inlet tube 122. The plurality of manifold openings 164 can each be connected to a corresponding opening in the plurality of openings 108. In some embodiments, the manifold can have different configurations where the number of manifold openings 164 is not equal to the plurality of openings 108. For example, FIG. 5C shows five manifold openings that correspond to five openings in distal end of the inlet tube 122. In some embodiments, a manifold could have three primary openings/branches (not shown) where two of the three openings/branches each divide into two additional openings/branches to match five openings in the wall of the inlet tube 122. Any combination of branches/sub-branches are possible in the manifold to achieve an arrangement similar to FIG. 5C.

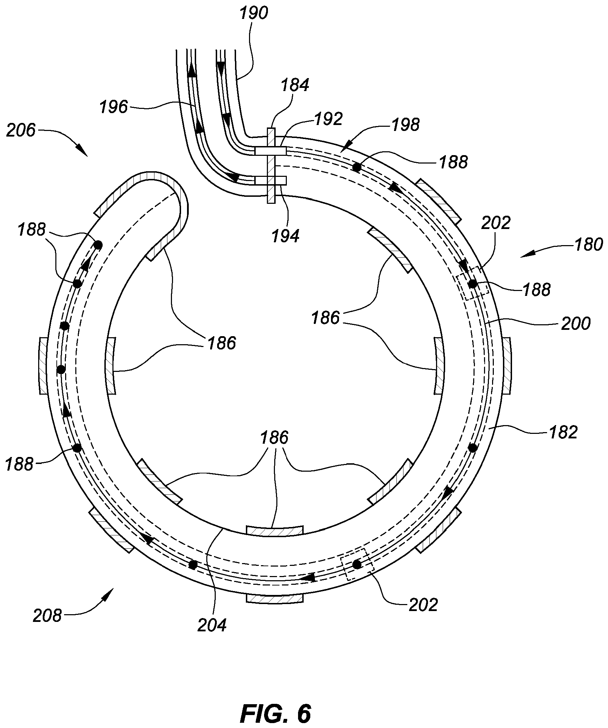

FIG. 6 is a cross-sectional view of a cryoablation catheter, consistent with embodiments of the present disclosure. The cryoablation catheter 180 can include an expansion chamber 182, an expansion chamber end wall 184, a plurality of electrodes 186, a plurality of openings 188, a supply line 190, an inlet 192, an outlet 194, and a circulation path 196.

The expansion chamber 182 can include an expansion chamber end wall 184 at a proximal end 198 of the expansion chamber 182. The expansion chamber end wall 184 can also include the inlet 192 and the outlet 194. The inlet 192 can be connected to an inlet tube 200. The inlet tube 200 can include a plurality of openings 188 at a plurality of locations. Each of a plurality of locations 202 can include a plurality of openings 188.

The plurality of locations 202 can be equally spaced (not shown) or the spacing can vary as shown in FIG. 6. The spacing of the plurality of locations 202 can facilitate coverage or patterns of coverage of the coolant as it is discharged from the plurality of openings 188. For example, the plurality of openings 188 can be arranged to direct the coolant at specific locations on the interior wall 204 of the expansion chamber 182 of the catheter 180. The arrangement of the plurality of openings 188 can be the same for each of the plurality of locations 202 or they can vary and different locations in the plurality of locations 202.

The discharge of coolant at the each of the plurality of locations 202 can be selectively controlled. For example, coolant may be discharged from only one location in the plurality of locations 202. In other embodiments, half of the locations can discharge coolant. The selective activation of the plurality of openings 188 at the plurality of locations 202 can be controlled by a controller (not shown). The controller can be part of, for example, the coolant control 32 and 62 shown in FIGS. 2 and 3A. The selective activation of the plurality of openings 188 can be controlled by any suitable method (valves, etc.). The selective activation controlled by the controller can vary, for example, a flow rate of the coolant through a particular opening, the length of time a particular opening is open, and/or the size of the particular opening.

The expansion chamber 182 can have a longitudinal center axis. The inlet tube 200 be offset from the longitudinal center axis (e.g., to accommodate a central lumen, etc.). In some embodiments, the inlet tube 200 can be aligned with the longitudinal center axis. The expansion chamber 182 can be any suitable size. For example, the expansion chamber 182 can be long enough to permit a distal end 206 of the catheter 180 to be formed into a hoop 208 (e.g., a length of the expansion chamber 182 (measured along the longitudinal center axis) can range between 35-104 mm) sized to fit various locations in a body as described herein.

The plurality of electrodes 186 can be, for example, ring electrodes. The plurality of electrodes 186 can be any suitable type of electrode. In some embodiments, multiple different types of electrodes are included in the plurality of electrode 186.

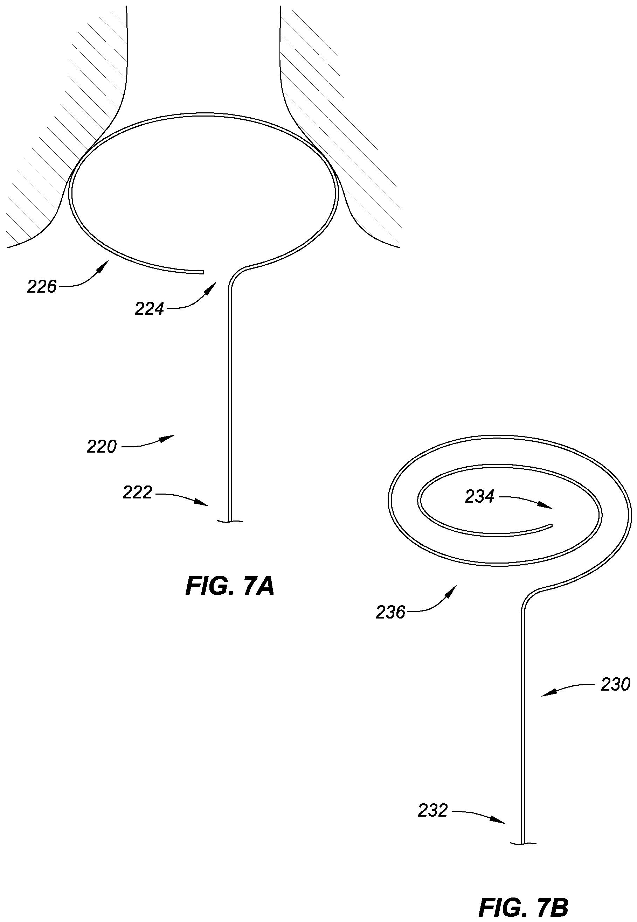

FIG. 7A is an isometric view of a cryoablation catheter with a hoop at a distal end, consistent with embodiments of the present disclosure. The cryoablation catheter 220 can have a proximal end 222 and a distal end 224. A portion of the distal end 224 can be formed into a hoop 226.

Similar to FIG. 3B above, the hoop 226 can be configured to fit, for example, into the wide antral portion of the PV. In other embodiments, the hoop 226 can be sized (e.g., smaller diameters) to fit further into the sleeve of the PV past the wider antral portion near the opening of the PV. The hoop 226 can include diameters ranging between, for example, 12-33 mm. The distal end 224 of the catheter 220 with the hoop 226 can be maneuvered adjacent to the PV using any suitable method and the hoop 226 can be maneuvered to a location of the PV.

FIG. 7B is an isometric view of a cryoablation catheter with multiple hoops at a distal end, consistent with embodiments of the present disclosure. A cryoablation catheter 230 can include a proximal end 232 and a distal end 234. A portion of the distal end 234 can be shaped into a multi-hoop configuration 236.

The multi-hoop configuration 236 can be configured, for example, with multiple hoops that are generally concentric. In the embodiment shown in FIG. 7B, the catheter 230 can be configured to form the multi-hoop configuration 236. The multiple-hoop configuration 236 can be arranged to facilitate coverage of, for example, planar surface inside a body. For example, the space between the portions of the multi-hoop configuration 236 can be sufficient to permit treatment, therapy, and/or monitoring to overlap (e.g., eliminate gaps). The multi-hoop configuration 236 can be formed using any suitable method (e.g., pull wires, shape memory material, etc.). In other embodiments, the catheter 230 can be configured into different shapes or patterns (e.g., an "S" or "Z" pattern, etc.) that can permit functionality similar to that of the multi-hoop configuration 236 (e.g., allowing portions of the catheter to be close enough to provide therapy/diagnostics to tissue without leaving gaps).

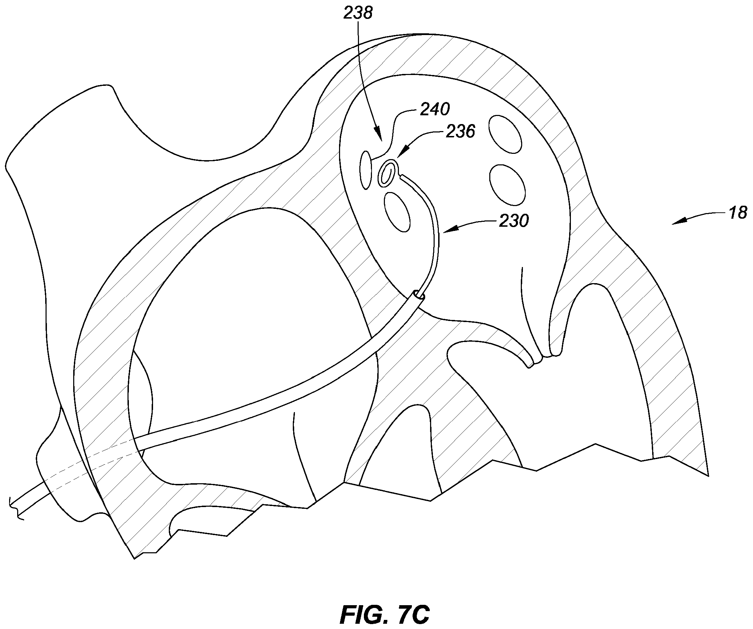

FIG. 7C is a cross-sectional front-view of a left atrium with the cryoablation catheter with multiple hoops of FIG. 7B positioned proximate a pulmonary vein in contact with tissue, consistent with various aspects of the present disclosure. The cryoablation catheter 230 can be used to position the cryoablation catheter with multiple hoops 236 to be in contact with tissue, for example, a heart wall 238 of a heart 18 (FIG. 1), proximate a pulmonary vein 240. The cryoablation catheter with multiple hoops 236 can be used where contact with tissue of a generally planar nature is needed (e.g., adjacent a pulmonary vein)

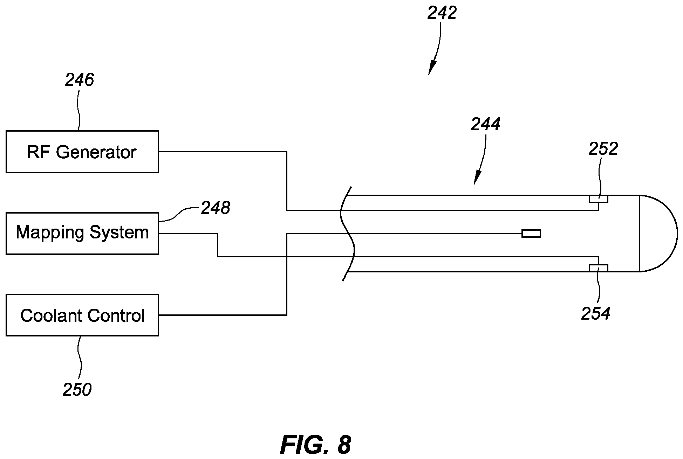

FIG. 8 is a schematic diagram of a cryoablation system, consistent with embodiments of the present disclosure. The cryoablation system 242 can include a catheter 244, a radio frequency (RF) generator 246, a mapping system 248, and a coolant control 250. The RF generator 246 can be electrically connected to, for example, an electrode 252. The mapping system 248 can be electrically connected to, for example, an electrode 254. In some embodiments, the RF generator 244 and the mapping system 248 can be electrically connected to the same electrode (not shown) or both the RF generator 246 and the mapping system 248 can be connected to the other electrode (e.g., the RF generator 246 and mapping system 248 connected to the electrode 252 and the RF generator 246 and mapping system 248 connected to the electrode 254). The coolant control 250 can be connected to a circulation loop (not shown) as described above and can, for example, circulate a coolant, control discharge of coolant into an expansion chamber (not shown), selectively activate one or more of a plurality of openings to discharge the coolant into the expansion chamber (not shown), and/or other similar functions.

The electrodes 252 and 254 can be any suitable electrode, such as a ring electrode and/or a tip electrode. The electrodes 252 and 254 can be used to provide therapy (e.g., RF ablation) and/or diagnostics (e.g., sensing or cardiac information). The same electrode can be used for multiple tasks at different times (e.g., the electrode 252 can first used for RF ablation, then used for sensing/diagnostic, etc.).

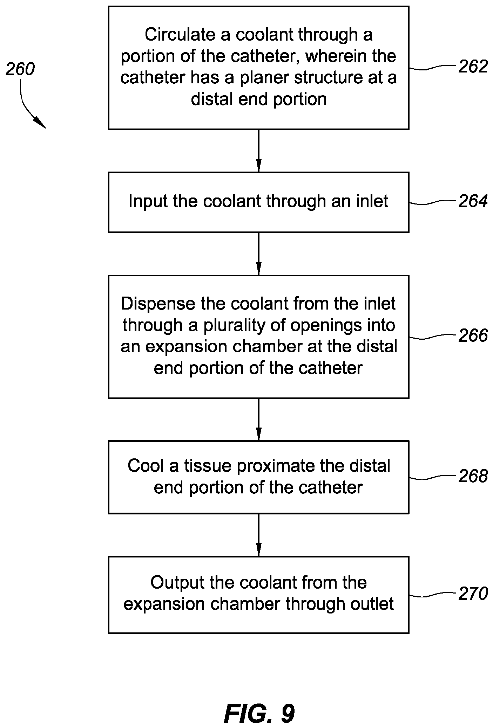

FIG. 9 shows an unclaimed exemplary method for circulating coolant through a portion of a catheter to cool tissue, consistent with embodiments of the present disclosure. A method 260 can include circulating a coolant through a portion of the catheter, wherein the catheter has a planar structure at a distal end portion at a block 262, inputting the coolant through an inlet at a block 264, dispensing the coolant from an inlet through a plurality of openings into an expansion chamber at a distal end of the catheter at a block 266, cooling the tissue proximate the distal end portion of the catheter at a block 268, outputting the coolant from the expansion chamber through an outlet, wherein a coolant supply line is coupled with the inlet, and the inlet is coupled with the plurality of openings and the outlet is coupled with the expansion chamber at a block 270.

As previously described above, the distal end of the catheter can be maneuvered to a location in the body using any suitable method (e.g., guide wires, pull wires, etc.). The catheter can be placed so that the distal end is proximate the tissue to be targeted (e.g., for therapy, for monitoring, etc.). In some embodiments the distal end can be adjacent to the tissue. In other embodiments a portion of the distal end can be in contact with the tissue.

The coolant (e.g., a liquid or a gas) can be circulated through a portion of the catheter. The catheter can be coupled with an inlet where the inlet is coupled with a plurality of openings. The plurality of openings can allow the coolant to circulate from the catheter to the expansion chamber. The coolant can cool the tissue proximate the distal end of the catheter.

After cooling the tissue, the coolant can be circulated from the expansion chamber through the outlet. In some embodiments, the coolant can be recirculated into the system for reuse (e.g., a closed loop circulation path that reuses the coolant). In other embodiments, the coolant can be discharged from the system after passing through the expansion chamber (e.g., an open loop circulation path that does not reuse the coolant).

Various embodiments are described herein to various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment is included in at least one embodiment. Thus, appearances of the phrases "in various embodiments," "in some embodiments," "in one embodiment," or "in an embodiment", or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

## Claims

1. An apparatus (92; 180) for providing therapy to tissue comprising
a flexible shaft with a distal end (90) and a proximal end, and
a planar therapy structure, wherein the planar therapy structure is coupled with the distal end (90) of the flexible shaft, wherein the planar therapy structure comprises
an expansion chamber (104; 182) coupled with the distal end (90) of the flexible shaft, comprising
an expansion chamber end wall (106; 184) at a proximal end of the expansion chamber (104, 182),
an inlet (94; 192) to receive a pressurized coolant from a supply line (132; 190), and
an outlet (96; 194) to receive a de-pressurized coolant from the expansion chamber (104; 182), and
a plurality of openings (108; 188) to provide the pressurized coolant to the expansion chamber (104; 182),
wherein the inlet (94; 192) and the outlet (96; 194) pass through the expansion chamber end wall (94; 192), and
the plurality of openings (108; 188) are coupled with the inlet (94; 192) and are inside the expansion chamber (104; 182).

2. The apparatus of claim 1, wherein the plurality of openings (108; 188) each have a corresponding size to control a flow rate of the coolant from the inlet (94; 192) into the expansion chamber (104; 182).

3. The apparatus of claim 1 or 2, wherein each of the plurality of openings (108; 188) further comprise a nozzle, wherein each nozzle is selectively activated by a controller (62).

4. The apparatus of any one of claims 1 to 3, wherein the flexible shaft further comprises a lumen (80, 82, 84, 86).

5. The apparatus of any one of claims 1 to 4, wherein the planar therapy structure further comprises a first wall portion (144, 146) with a first cross-section and a first thermal conductivity and a second wall portion (144, 146) with a second cross-section and a second thermal conductivity where the first and second cross-sections are different and the first and second thermal conductivities are equal.

6. The apparatus of any one of claims 1 to 5, wherein the planar structure is a hoop.

7. The apparatus of any one of claims 1 to 6, wherein the planar structure is a multi-hoop configuration.

8. The apparatus any one of claims 1 to 7, wherein the apparatus further comprises a plurality of electrodes (102; 186).

9. A system comprising
a coolant source (64), wherein the coolant source generates a pressurized coolant,
a coolant control (62), wherein the coolant control controls the coolant source and the pressurized coolant,
the apparatus of any one of claims 1 to 8, wherein the apparatus is a catheter, wherein the catheter is coupled with the coolant source and the catheter comprises
the planar therapy structure, where the planar therapy structure is coupled with the distal end (90) of the shaft, wherein
the inlet is configured to receive the pressurized coolant from the coolant source.

10. The system of claim 9, wherein the system further comprises a mapping system for generating a map of a location in a body.

11. The system of claim 9 or 10, wherein the system further comprises an ablation system for ablating tissue, wherein the ablation system comprises a signal generator and an electrode coupled with a distal end portion of the catheter.

12. The system of claim 9, 10 or 11, wherein the catheter further comprises an electrode (102; 186), wherein the electrode is located proximate the distal end.

13. The system of claim 12, wherein the electrode detects cardiac signals and delivers ablation energy.

14. The system of any one of claims 9 to 13, wherein the plurality of openings (108; 188) are selectively activated to control a flow of the coolant from the inlet (94; 192) into the expansion chamber (104; 182).

## Patentansprüche

1. Vorrichtung (92; 180) zum Vorsehen einer Therapie an einem Gewebe, mit
einem flexiblen Schaft mit einem distalen Ende (90) und einem proximalen Ende, und
einer planaren Therapiestruktur, bei der die planare Therapiestruktur mit dem distalen Ende (90) des flexiblen Schaftes gekoppelt ist, bei der die planare Therapiestruktur
eine Ausdehnungskammer (104; 182), die mit dem distalen Ende (90) des flexiblen Schaftes gekoppelt ist, und
eine Ausdehnungskammerendwand (106; 184) an einem proximalen Ende der Ausdehnungskammer (104; 182) aufweist,
einen Einlass (94; 192) zum Aufnehmen eines druckbeaufschlagten Kühlmittels von einer Zufuhrleitung (132; 190), und
einen Auslass (96; 194) zum Empfangen eines druckentlasteten Kühlmittels von der Ausdehnungskammer (104; 182) aufweist, und
eine Mehrzahl von Öffnungen (108; 188) zum Vorsehen des unter Druck stehenden Kühlmittels an die Ausdehnungskammer (104; 184) aufweist,
bei der der Einlass (94; 192) und der Auslass (96; 194) durch die Ausdehnungskammerendwand (94; 192) passieren, und
die Mehrzahl von Öffnungen (108; 188) mit dem Einlass (94; 192) gekoppelt sind und im Inneren der Ausdehnungskammer (104; 182) sind.

2. Vorrichtung nach Anspruch 1, bei der jede der Mehrzahl von Öffnungen (108; 188) eine entsprechende Größe zum Steuern einer Strömungsrate des Kühlmittels von dem Einlass (94; 192) in die Ausdehnungskammer (104; 182) aufweist.

3. Vorrichtung nach Anspruch 1 oder 2, bei der jede der Mehrzahl von Öffnungen (108; 188) ferner einen Stutzen aufweist, bei der jeder Stutzen selektiv durch eine Steuerung (62) aktiviert wird.

4. Vorrichtung nach einem der Ansprüche 1 bis 3, bei der der flexible Schaft ferner einen Lumen (80; 82; 84; 86) aufweist.

5. Vorrichtung nach einem der Ansprüche 1 bis 4, bei dem die planare Therapiestruktur ferner einen ersten Wandbereich (144, 146) mit einem ersten Querschnitt und einer ersten thermischen Leitfähigkeit und einem zweiten Wandbereich (144, 146) mit einem zweiten Querschnitt und einer zweiten Leitfähigkeit aufweist, wobei der erste und der zweite Querschnitt unterschiedlich sind und die erste und die zweite Leitfähigkeit gleich sind.

6. Vorrichtung nach einem der Ansprüche 1 bis 5, bei der die planare Struktur eine Schlaufe ist.

7. Vorrichtung nach einem der Ansprüche 1 bis 6, bei der die planare Struktur eine Mehrfachschlaufkonfiguration ist.

8. Vorrichtung nach einem der Ansprüche 1 bis 7, bei der die Vorrichtung ferner eine Mehrzahl von Elektroden (102; 186) aufweist.

9. System, mit
einer Kühlmittelquelle (64), bei dem die Kühlmittelquelle ein druckbeaufschlagtes Kühlmittel erzeugt,
einer Kühlmittelsteuerung (62), bei der die Kühlmittelsteuerung die Kühlmittelquelle und das druckbeaufschlagte Kühlmittel steuert,
der Vorrichtung nach einem der Ansprüche 1 bis 8, bei dem die Vorrichtung ein Katheter ist, und der Katheter mit der Kühlmittelquelle gekoppelt ist und der Katheter
die planare Therapiestruktur aufweist, wobei die planare Therapiestruktur mit dem distalen Ende des Schaftes (90) gekoppelt ist, bei dem
der Einlass dazu konfiguriert ist, das druckbeaufschlagte Kühlmittel von der Kühlmittelquelle zu empfangen.

10. System nach Anspruch 9, bei dem das System ferner ein Abbildungssystem zum Erzeugen einer Abbildung einer Stelle in einem Körper aufweist.

11. System nach Anspruch 9 oder 10, bei dem das System ferner ein Ablationssystem zur Ablation von Gewebe aufweist, bei dem das Ablationssystem einen Signalgenerator und eine Elektrode aufweist, die mit einem distalen Endbereich des Katheters gekoppelt ist.

12. System nach Anspruch 9, 10 oder 11, bei dem der Katheter ferner eine Elektrode (102; 186) aufweist, bei dem sich die Elektrode benachbart dem distalen Ende befindet.

13. System nach Anspruch 12, bei dem die Elektrode kardiologische Signale erfasst und eine Ablationsenergie zuführt.

14. System nach einem der Ansprüche 8 bis 13, bei dem die Mehrzahl von Öffnungen (108; 188) selektiv zum Steuern einer Strömung des Kühlmittels von dem Einlass (94; 192) in die Ausdehnungskammer (104; 182) aktiviert werden.

## Revendications

1. Appareil (92 ; 180) pour fournir une thérapie à un tissu comprenant
une tige flexible avec une extrémité distale (90) et une extrémité proximale, et
une structure de thérapie planaire, dans lequel la structure de thérapie planaire est couplée à l'extrémité distale (90) de la tige flexible, dans lequel la structure de thérapie planaire comprend
une chambre d'expansion (104 ; 182) couplée à l'extrémité distale (90) de la tige flexible, comprenant
une paroi d'extrémité de chambre d'expansion (106; 184) à une extrémité proximale de la chambre d'expansion (104, 182),
une entrée (94 ; 192) pour recevoir un liquide de refroidissement sous pression à partir d'une ligne d'alimentation (132 ; 190), et
une sortie (96 ; 194) pour recevoir un liquide de refroidissement dépressurisé de la chambre d'expansion (104 ; 182), et
une pluralité d'ouvertures (108; 188) pour fournir le liquide de refroidissement sous pression à la chambre d'expansion (104 ; 182),
dans lequel l'entrée (94 ; 192) et la sortie (96 ; 194) passent à travers la paroi d'extrémité de chambre d'expansion (94 ; 192), et
la pluralité d'ouvertures (108 ; 188) sont couplées avec l'entrée (94 ; 192) et
sont à l'intérieur de la chambre d'expansion (104 ; 182).

2. Appareil selon la revendication 1, dans lequel la pluralité d'ouvertures (108 ; 188) ont chacune une taille correspondante pour contrôler un débit du liquide de refroidissement depuis l'entrée (94 ; 192) dans la chambre d'expansion (104 ; 182).

3. Appareil selon la revendication 1 ou 2, dans lequel chacune de la pluralité d'ouvertures (108 ; 188) comprend en outre une buse, dans lequel chaque buse est activée de manière sélective par un contrôleur (62).

4. Appareil selon l'une quelconque des revendications 1 à 3, dans lequel la tige flexible comprend en outre une lumière (80, 82, 84, 86).

5. Appareil selon l'une quelconque des revendications 1 à 4, dans lequel la structure de thérapie planaire comprend en outre une première partie de paroi (144, 146) avec une première section transversale et une première conductivité thermique et une seconde partie de paroi (144, 146) avec une seconde section transversale et une seconde conductivité thermique où les première et seconde sections transversales sont différentes et les première et seconde conductivités thermiques sont égales.

6. Appareil selon l'une quelconque des revendications 1 à 5, dans lequel la structure planaire est un cerceau.

7. Appareil selon l'une quelconque des revendications 1 à 6, dans lequel la structure planaire est une configuration multi-cerceau.

8. Appareil selon l'une quelconque des revendications 1 à 7, dans lequel l'appareil comprend en outre une pluralité d'électrodes (102 ; 186).

9. Système comprenant
une source de liquide de refroidissement (64), dans lequel la source de liquide de refroidissement génère un liquide de refroidissement sous pression,
un contrôle de liquide de refroidissement (62), dans lequel le contrôle de liquide de refroidissement contrôle la source de liquide de refroidissement et le liquide de refroidissement sous pression,
l'appareil selon l'une quelconque des revendications 1 à 8, dans lequel l'appareil est un cathéter, dans lequel le cathéter est couplé à la source de liquide de refroidissement et le cathéter comporte
la structure de thérapie planaire, où la structure de thérapie planaire est couplée à l'extrémité distale (90) de la tige, dans lequel
l'entrée est configurée pour recevoir le liquide de refroidissement sous pression de la source de liquide de refroidissement.

10. Système selon la revendication 9, dans lequel le système comprend en outre un système de cartographie pour générer une carte d'un emplacement dans un corps.

11. Système selon la revendication 9 ou 10, dans lequel le système comprend en outre un système d'ablation pour l'ablation de tissu, dans lequel le système d'ablation comprend un générateur de signaux et une électrode couplée à une partie d'extrémité distale du cathéter.

12. Système selon la revendication 9, 10 ou 11, dans lequel le cathéter comprend en outre une électrode (102 ; 186), dans lequel l'électrode est située à proximité de l'extrémité distale.

13. Système selon la revendication 12, dans lequel l'électrode détecte des signaux cardiaques et délivre une énergie d'ablation.

14. Système selon l'une quelconque des revendications 9 à 13, dans lequel la pluralité d'ouvertures (108 ; 188) sont sélectivement activées pour contrôler un écoulement du liquide de refroidissement depuis l'entrée (94 ; 192) dans la chambre d'expansion (104 ; 182).
